# EUROPEAN PATENT APPLICATION

(11) **EP 3 530 263 A1**
(43) Date of publication of application: **28.08.2019**
(21) Application number: 19159300.3
(22) Date of filing: 26.02.2019
(51) Int. Cl.: A61K 8/362, A61K 8/365, A61K 8/46, A61Q 5/00, A61Q 5/04, A61Q 5/06

(54) **COSMETIC TREATMENT FOR THE IMPROVEMENT OF THE ESTHETIC ASPECT AND PHYSICAL-MECHANICAL CHARACTERISTICS OF KERATINS USING SULFO-CARBOXYLATE MOLECULAR PINCERS**

(30) Priority: 26.02.2018 IT 201800003035
(71) Applicant: Jean Paul Myne' Srl, 62100 Macerata (IT)
(72) Inventor: MANNOZZI, Alderano, 63100 Ascoli Piceno (IT)
(74) Representative: Primiceri, Maria Vittoria

(57) **Abstract**

This invention concerns treatment of keratins, and in particular of the keratins in human hair and other animal fur. The invention, in particular, concerns a method for improving the esthetic aspect and physical-mechanical characteristics of them through the application on them of organic binders provided with carboxylics and/or sulfonics capable of forming new covalent chemical bonds with the primary aminic functional groups of some of the amino acids that are part of the primary structure of the keratins (substances defined also "molecular pincers").

## Description

### Field of the invention

This invention concerns treatment of keratins, and in particular of the keratins in human hair and other animal fur. The invention, in particular, concerns a method for improving the esthetic aspect and physical-mechanical characteristics of them through the application on them of organic binders provided with carboxylics and/or sulfonics capable of forming new covalent chemical bonds with the primary aminic functional groups of some of the amino acids that are part of the primary structure of the keratins (substances defined in the text of the patent application also with the words "molecular pincers").

### Background of the invention

According to common knowledge at the current state of the art, keratins are a group of proteins with a fibrous structure and, generally, a high content of sulfur, which are created by living cells as expression of their genetic code (DNA). These proteins form fibrous macro-aggregates as hair, nails and the surface layers of the skin. Keratins are classified by type as alpha-, beta-, and amorphous.

The structure of keratins, their esthetic, chemical-physical and mechanical characteristics and the techniques for altering their structure to improve the appearance and cosmetic performance are reflected in the state of the art by the documents:
- Boga C. et al.: Curly or smooth? Old and new answers - Household and Personal Care Today: Hair care monographic supplement Vol. 10 (2) - March/April 2015;
- Istrate D.V.: Thesis on Heat Induced denaturation of fibrous hard α-keratins and their reaction with various chemical reagents, 2011;
- Quadflieg J.M.: Thesis on Fundamental properties of afro-american hair as related to their straightening / relaxing behavior, 2003;
- Robbins C.R.: Chemical and Physical Behavior of Human Hair, 2012;
- Tate M.L. et al.: Quantification and prevention of hair damage J. Soc. Cosmet. Chem., 1993, 44, 347-3 71 TRI/Princeton, Princeton, NJ-USA;
- Wang B.: Keratin: Structure, mechanical properties, occurrence in biological organisms and efforts at bioinspiration, Progress in Materials Science, Elsevier Ltd. - 2016 76 229-318.

From these documents it can be seen that the concentration in natural keratins of amino acids with residual acid (Aspartic Acid and Glutamic Acid) or basic (Lysine, Histidine and Arginine) functional groups is similar, and in some cases greater, than the content in Cysteine and/or Cystine.

In keratins that have undergone treatments of bleaching, waving, dyeing, straightening or relaxing, the concentration of cysteic acid can be as great or even greater than the concentration of Cysteine.

On the other hand, in keratins that have undergone the above treatments, the concentration of thiolic groups (R-SH) is absolutely negligible (0.01-0.1 % of total amino acids) except in the case of waving where, only in the reduction stage with thioglycolates or other substances with suitable redox potential, high concentrations of thiolic groups are formed.

These, however, in the subsequent stage of treatment, are re-oxidated to disulfide bonds by oxidants such as hydrogen peroxide, sodium bromate or the like.

The document WO2016100634 A2 discloses a composition for hair conditioning inclusive of one or more materials useful for improving the fibrous properties of the hair. In particular, the composition underlying the treatment proposed by document WO2016100634 A2 has the scope of reintegrating the natural lipids that have an important role in the structure of the hair, where they bind the proteins that are an integral part of the cuticular membrane/outer layer of the hair.

Document WO2016074986 A1 discloses a composition to be used as a shampoo or conditioner that untangles the hair making it easier to comb. In particular, the composition proposed calls for the use of hydrophilic solubilizing substances, cationic polymers and surfactant, excluding the use of hydrophobic substances such as natural/synthetic oils and/or silicones.

Furthermore, in the state of the art the following documents are known:
- FR 2779642 A1 discloses:
   - a cosmetic composition for treating hair keratin fibers, comprising at least one branched sulphonic polyester and at least one conditioning agent chosen from the group comprising non-volatile silicones, cationic or amphoteric polymers and cationic or amphiphilic surfactants;
   - the use of a branched sulphonic polyester in order to obtain better performance in the cosmetic treatment of hair keratin fibers.
- US 2017/151144 A1 discloses a cosmetic composition for hair treatment comprising at least one mono or bi functional organic acid with respectively one or two carboxylic functional groups -COOH.
- FR 3001385 A1 discloses a cosmetic composition for hair treatment comprising an organic dicarboxylic acid, whose structure contains from 2 to 8 carbon atoms and a vegetable oil with a fraction of oleic acid not higher than 20% by weight.
- WO 2010/049434 A2 discloses a cosmetic composition for hair treatment comprising at least one mono, bi or trifunctional organic acid, each having one, two or three carboxylic functional groups -COOH, or comprising a mixture thereof.

The cosmetic action claimed in document FR 2 779 642 A1 is carried out only by the branched sulphonic polyester, which does not contain carboxyl functional groups -COOH but only anionic sulfonate functional groups -SO₃- and -COOR' ester functional groups.

In the remaining three documents no reference is made to a specific reaction mechanism between mono, bi or tri functional carboxylic acids of the claimed cosmetic compositions and hair keratins. The use of such compositions at basic or strongly basic pH and the presence in all compositions of alkali and/or alkaline-earth metal salts, confirms the realization of a ionic reaction mechanism which involves the carboxylated anionic functional groups -COO- and not, rather, the realization of a covalent-type mechanism between the carboxyl groups -COOH of the molecular pincers and the free primary amino groups of the keratins.

### Disclosure of the invention

The purpose of this invention is to provide a method for the treatment of keratins that is able to repair and/or improve the structure of keratins damaged by previous cosmetic treatments, or to improve the aspect and physical-mechanical characteristics of keratins that have not been treated cosmetically.

Another purpose of this invention is to provide a method for the treatment of keratins that is able, through the formation of new covalent chemical bonds defined as molecular pincers, to improve the structure of the keratins themselves.

These and other purposes will be achieved with this invention, which concerns a method for the treatment of the keratins with organic binders containing carboxylic and/or sulfonic functional groups.

### Detailed description of the invention

According to a preferred - but not exclusive - embodiment, this invention concerns a method for the treatment of keratins with organic binders containing carboxylic and/or sulfonic functional groups.

The invention consists of the use of a family of organic substances containing carboxylic and/or sulfonic functional groups (defined as molecular pincers for their ability to form new covalent chemical bonds with the primary aminic functional groups naturally present in the lateral chains of the amino acids Arginine, Lisine and Istidine), in order to improve the esthetic aspect and the physical-mechanical characteristics of keratins.

The organic binders used as molecular pincers are characterized by the chemical structure indicated hereafter (Formula I): 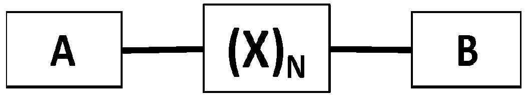 where:
- A and B are reactive molecular portions independently containing carboxylic and/or sulfonic functional groups, capable of reacting with the free primary aminic functional groups of keratins to form new imidic functional groups;
- N is a whole number between 0 and 100, preferably between 0 and 50, more preferably between 0 and 10;
- X can be a single atom, a group of atoms, as well as a functional group (such as -C(=O)-, -CH₂-), or several groups of atoms, such as an alkylenic or polymeric chain. "X" includes, but is not limited to Sulfur, Carbon, Boron, Nitrogen and alcoxic, alkylic, alkenylic, cycloalkylic, cycloalkenylic, arylic, heterocycloalkylic compounds or ether.

The following are some examples of binders: Glutaric acid (CAS no. 110-94-1), Succinic acid (CAS 110-15-6), Malic acid (CAS 97-67-6), Phthalic acids (ortho, metha, para) (CAS no. 88-99-3, 121-91-5, 100-12-0), spermidic acid (CAS no. 4386-03-2), 2-sulfur-benzoic acid (CAS no. 632-25-7), methane-disulfonic acid (CAS no. 503-40-2), butane-1.4 disulfonic acid (CAS no. 27665-39-0).

In consideration of their particular three-dimensional structure (at least two reactive functional groups spaced by an inert organic skeleton for the purpose of the reaction), the organic binders that are the subject of this patent application can be compared to molecular pincers capable of altering the secondary, tertiary or quaternary structures of the keratinic proteins through the formation of new covalent chemical bonds.

The organic binders that are the subject of this patent application, containing carboxylic and/or sulfonic functional groups, are able to react with at least one aminic functional group of keratins by forming covalent chemical bonds that lead to the creation of new functional groups belonging to the family of the imides, performing the function of new "molecular bridges" between filaments of keratins or within the keratinic fiber itself.

The new imidic functional groups, or new "molecular bridges" may form, depending on the chemical structure of the substances used, in points that are neither topographically adjacent to nor contiguous with the keratinic filaments.

These new functional groups, depending on their position in space, can alter the structure of the keratins in more or less marked ways, changing the configuration and the physical-mechanical characteristics, with the result of improving the esthetic aspect and physical-mechanical characteristics of both the keratinic fibers and their agglomerates (hair, or animal fur).

These organic binders can be used dissolved or dispersed in a "cosmetically acceptable medium" (meaning by this a mixture of substances commonly used in the manufacture of cosmetic products) in a concentration between 0.1% and 99.9% by weight/weight.

The substances commonly used in the manufacture of cosmetic products are to be understood as those listed in the International Cosmetic Ingredient Dictionary and Handbook Sixteenth Edition 2016 5 volumes, published periodically by the American organization "Personal Care Products Council" 1620 L Street, NW Washington, D.C.).

They are classified in categories including, but not limited to, the following: abrasive, absorbent, anti-agglomerant, anticorrosive, antidandruff, defoamer, antimicrobic, antioxidant, anti-perspirant, anti-plaque, antiseborrheic, antistatic, astringent, binder, whitener, buffer, swelling agent, chelator, detergent, cosmetic dye, denaturant, deodorant, depilatory, disentangler, emollient, emulsifier, emulsion stabilizer, film coating agent, fragrance, foamer, gelling agent, hair conditioner, hair dye, hair conditioner, hair color, fixative for hair, waving or straightening agent for hair, moistener, hydrotrope, keratolith, masking agent, moisturizer, nail conditioner, matting agent, oxidizer, pearlescent, fragrance, plastifier, preservative, propellant, reducing agent, filler, freshener, skin conditioner, protective for the skin, soluble smoothing agent, soothing stabilizer, surfactant, tanning agent, tonic, UV absorbent, UV filter, viscosity regulator.

More specifically, we indicate three different possible situations that may arise using the organic binders described above:

### 1. Formation of sulfo-carboxy-imidic functional groups by using:

- **1.a** organic binders containing a carboxylic and a sulfonic functional group in their molecular structure, capable of reacting with primary aminic functional groups of two distinct fibers of keratin (extra-chain reaction) or of the same keratin fiber (intra-chain reaction).
   The term "sulfo-carbox-imidic" is used here to mean a set of chemical bonds of the covalent type between atoms of Carbon, Sulfur and Nitrogen according to the structure:
   (-S(=O)₂-N(R)-C(=O)- where (R)- indicates the lateral chain of amino acids Lisine, Arginine and Istidine and the groups C=O and SO₂ that, independently, belong to the organic binder.
   The figures below show the reactions possible between the reactive functional groups of the organic binders indicated in the preceding section (1.a) and the keratinic fibers.
   **Fig. A:** Formation of sulfo-carboxy-imidic functional groups according to the process described above section 1.a (extra-chain keratinic reaction) 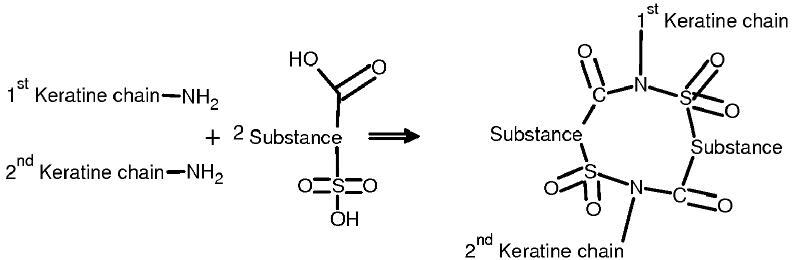
   **Fig. B:** Formation of sulfo-carboxy-imidic functional groups according to the process described above section 1.a (intra-chain keratinic reaction)

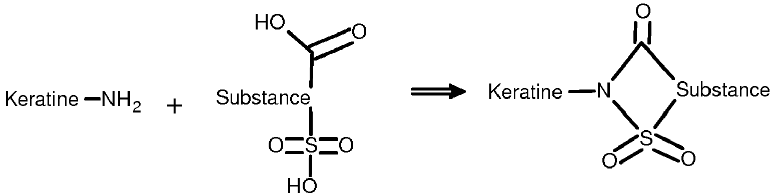

### 2. Formation of disulfo-imidic functional groups by using:

- **2.a** organic binders containing two sulfonic functional groups in their molecular structure, capable of reacting with primary aminic functional groups of two distinct fibers of keratin (extra-chain reaction) or of the same keratin fiber (intra-chain reaction).
   The term "disulfo-imidic" is used here to mean a set of chemical bonds of the covalent type between atoms of Sulfur and Nitrogen according to the structure:
   (-S(=O)₂-N(R)-S(=O)₂-) where (R)- indicates the lateral chain of amino acids Lisine, Arginine and Istidine and the SO₂ groups that belong to the organic binder.
   The figures below show the reactions possible between the reactive functional groups of the organic binders indicated in the preceding section 2.a and keratinic fibers.
   **Fig. C:** Formation of disulfo-imidic functional groups according to the process described above section 2.a (extra-chain keratinic reaction) 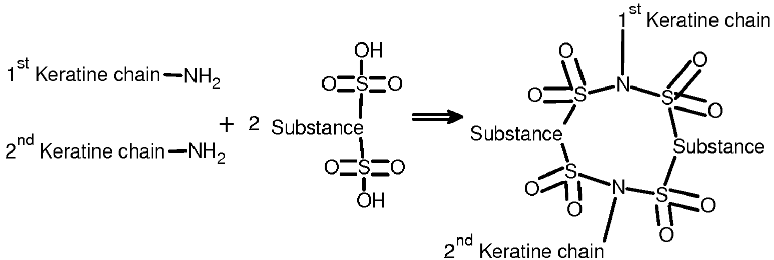
   **Fig. D:** Formation of disulfo-imidic functional groups according to the process described above section 2.a (intra-chain keratinic reaction)

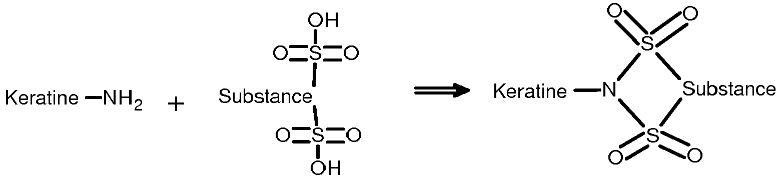

### 3. Formation of dicarboxy-imidic functional groups by using:

**3.a** organic binders containing two carboxylic functional groups in their molecular structure, capable of reacting with primary aminic functional groups of two distinct fibers of keratin (extra-chain reaction) or of the same keratin fiber (intra-chain reaction);
The term "dicarboxy-imidic" is used here to mean a set of chemical bonds of the covalent type between atoms of Carbon and Nitrogen according to the structure:
(-C(=O)-N(R)-C(=O)-) where (R)- indicates the lateral chain of amino acids Lisine, Arginine and Istidine and the C=O groups that belong to the organic binder.
The figures below show the reactions possible between the reactive functional groups of the organic binders indicated in the preceding section (3.a) and the keratinic fibers.
**Fig. E:** Formation of dicarboxy-imidic functional groups according to the process described above section 3.a (extra-chain keratinic reaction) 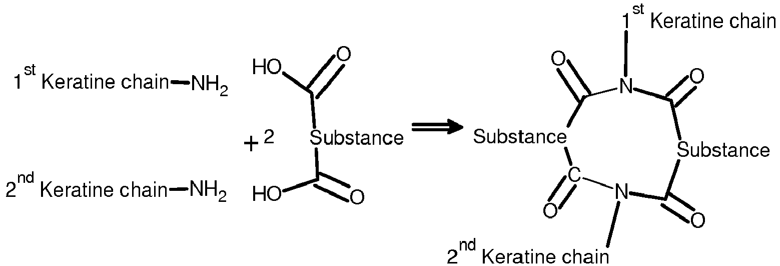
**Fig. F:** Formation of dicarboxy-imidic functional groups according to the process described above section 3.a (intra-chain keratinic reaction)

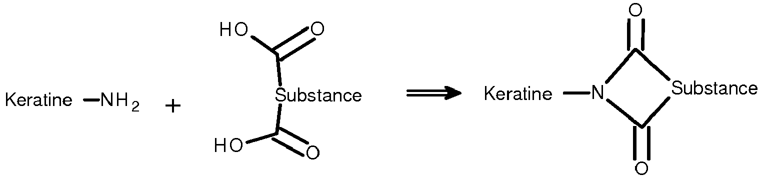

### EXAMPLES OF APPLICATION:

On the basis of the hypotheses formulated, a number of tests were carried out using some of the substances belonging to the family of organic binders, and the results obtained are described in the following examples.

### Example 1: Comparison between the traditional permanent wave and a permanent wave performed using the organic binder butane-1,4 disulfonic acid (CAS n. 27665-39-0).

Samples of hair were obtained from a human subject and cut in two locks of about 1 cm width and weight of about 5 grams.

Commercial kit for permanents: the kit used, produced by Cosmec srl of Milan (Italy), contained in step 1 the reducing substances based on thioglycolates of ammonium dispersed in a "cosmetically acceptable medium" and, as oxidizing agent (step 2) hydrogen peroxide, also dispersed in a "cosmetically acceptable medium".

Organic binder used: Butane-1,4 disulfonic acid (CAS n. 27665-39-0) at a concentration of 0.5 g dissolved in 9.5 g water (binding mixture).

The hair was washed with shampoo, dried with a towel and then wound around a curler for permanents (a tool with which persons expert in the sector is thoroughly familiar).

Step 1 of the commercial kit was applied on the two locks of hair and left in place for 20 minutes (during this time the disulfur bridges (-S-S-) of the cystine "open" to form sulfhydrilic or thiolithic (-SH) cysteine groups). The hair was then rinsed with warm water and dried with a towel.

### Method using organic binder

The binding mixture was applied to the first lock of hair after wetting the hair, and left in place for 20 minutes.

The lock of hair was then rinsed with warm water for about 1-2 minutes and unrolled from the curler.

The lock was dehydrated by heating it with a special hair curling iron (with which experts in the sector are familiar) at the temperature of 150°C.

The lock was washed with Keratin Plus shampoo from the Treasure Jean Paul Myne'® line.

The washing and drying steps were repeated 10 times.

### Method without using organic binder

A second lock of hair was treated as described above, except that, instead of the organic binder, step 2 of the commercial kit (containing hydrogen peroxide) was used, and applied in the same way as the organic binder, for a contact time of 20 minutes.

### Results

Both permanents (using the binder formulation or the hydrogen peroxide) exhibited good resistance of the wave produced by the permanent after a first washing and conditioning. After 10 cycles of washing and drying with the same shampoo and conditioner, a marked reduction of the wave compared with the situation obtained after the first washing with shampoo and conditioner was observed.

However, the aspect and consistency of the permanent obtained using the binding formulation showed that the hair was shinier and less kinky than the permanent obtained using only the commercial kit.

The lock treated with the organic binder exhibited a significant difference in tensile strength and sheen compared to the lock treated with the commercial kit without the organic binder.

### Example 2: Control of sheen and consistency of hair after treatment with binding formulation.

A sample of natural (untreated) gray hair was obtained from a human subject.

Organic binder used: Spermidic acid (CAS no. 4386-03-2) 0.5 g dissolved in 9.5 g water (binding mixture). The resulting solution was mixed with Jean Paul Myné® Ashana conditioner in a ratio of 1:4.

### Methods

A lock of natural gray hair was washed with Jean Paul Myné® Ashana shampoo and dried with a towel.

The hair was combed using a comb with wide teeth, then using one with fine teeth for 2 minutes.

After combing, the binding mixture (about 5 ml) was applied manually, using a brush, to the sample of hair which was then combed for about 1 minute.

The hair sample was left untouched for about 10 minutes, then rinsed with water, then washed again with Jean Paul Myné® Ashana Shampoo and Conditioner.

The same hair sample was washed and conditioned five (5) more times with the same shampoo and conditioner.

A second section of the virgin gray hair (the control) was treated exactly as described above, but the binding mixture was not applied to that hair sample.

The locks were dehydrated by heating with a special hair curling iron (with which experts in the sector are familiar) at the temperature of 180°C.

### Results

The samples of hair treated with the binding formulation were shinier and felt softer to the touch than the control sample not treated with the binding mixture.

The lock treated with the organic binder exhibited a significant difference in tensile strength and sheen compared to the lock treated with the commercial kit without the organic binder.

The sample of hair treated with the binding mixture had a "healthier" overall esthetic aspect than the control sample. The sheen, consistency and general aspect remained unchanged after five washings with shampoo and conditioner.

### Example 3: Control of sheen and consistency of bleached hair after treatment with binding formulation.

A sample of virgin blond hair, untreated, described by experts in the sector as highly porous and difficult to comb, was obtained from a human subject. Organic binder used: Succinic acid (CAS no. 110-15-6) 0.5 g dissolved in 9.5 g water (binding mixture).

The resulting solution was mixed with Jean Paul Myné® Ashana conditioner in a ratio of 1:4.

### Methods

A lock of untreated blond hair was washed with Jean Paul Myné® Ashana shampoo and dried with a towel. The hair was combed using a comb with wide teeth, followed by combing with fine tooth comb for 5 minutes.

The binding mixture (about 5 ml) was then applied with a brush manually on the lock of hair and it was combed for about 2 minutes.

The hair sample was left undisturbed for about 10 minutes, after which it was treated again with the binding formulation (about 5 ml).

The hair sample was combed for about 10 seconds and left undisturbed for about 5 minutes.

The hair sample was then rinsed with water, and washed with Jean Paul Myné® Ashana shampoo and conditioner before being examined.

After the initial examination, the sample was washed and conditioned two (2) more times with the same shampoo and conditioner.

A second section of untreated blond hair (the control) was treated exactly as described above, except for the fact that the binding mixture was not applied.

Then, after combing this lock of hair and washing it with Jean Paul Myné® Ashana shampoo, the conditioner Jean Paul Myné® Ashana was applied (without the binding mixture).

The locks were dehydrated by heating with a special hair curling iron (with which experts in the sector are familiar) at the temperature of 180°C.

### Results

The sample of hair treated with the binding formulation were shinier and felt softer to the touch than the control sample not treated with the binding mixture.

The treated sample of hair had a healthier overall esthetic aspect than the control sample.

The sheen, consistency and general aspect remained unchanged after two treatments with shampoo and conditioner.

The lock treated with the organic binder exhibited a significant difference in tensile strength and sheen compared to the lock treated with the commercial kit without the organic binder.

### Example 4: Check of color retention and consistency of colored hair treated with the binding formulation.

Three samples of hair about 1 cm wide and weighing about 5 grams each (locks) were obtained from a human subject.

Dye formulation: the formulation used to color the hair was obtained from a hair color service of Jean Paul Myné® DOC color #10 (40 grams) + Jean Paul Mynè® DOC ENZYME 0.3 (hydrogen peroxide 30 volumes). Organic binder used: Malic acid (CAS no. 97-67-6) 0.5 g as a concentration of 0.5 g in 9.5 g water (binding mixture).

### Methods

The hair samples were washed with a shampoo Jean Paul Myné® Treasure Enhancing then dried with a towel.

The samples were then dyed with the hair color service of Jean Paul Myné® DOC color indicated above for a contact time of 30 minutes.

The binding mixture was applied to the hair samples of the second and third lock using a brush and massaging with the fingers to facilitate absorption of the binding mixture by the keratinic fibers.

The binding mixture was then left on the second hair sample for about 1 minute and on the third sample for about 10 minutes.

The hair samples were then rinsed and washed with Jean Paul Myné® Treasure Enhancing Shampoo and Conditioner five times before being examined.

### Results

The samples of hair treated with the binding mixture showed better color retention and were smoother to the touch, with less of the kinky effect than the control sample.

The samples of hair treated with the binding mixture had a healthier overall esthetic aspect than the control sample.

The third lock, which had been left in contact with the binding mixture for 10 minutes showed a significant different in sheen with respect to the lock exposed to the binding mixture for 1 minute.

The locks treated with the organic binder showed a significant difference in tensile strength with respect to the lock treated without the addition of the organic binder.

### Example 5: comparison between hair treated with a bleaching cosmetic product applied simultaneously with the binding mixture and hair treated with the bleaching cosmetic product alone.

The binder used in this example was the mixture used in example 1 (Butane-1,4 disulfonic acid - CAS n. 27665-39-0) in the concentration of 1 gram dissolved in 9 grams of water.

Two samples of human hair taken from the same head were tested. They were 1 cm wide and about 20 cm long and weighed 5 grams each.

The color was medium brown (grade 4) and they had been previously colored with an unknown professional hair dye.

**Sample 1:** the sample was bleached with Jean Paul Myné® DOC OLEODEC 30 grams + DOC ENZYME 0.3 30 grams + 7 grams of binding mixture, all blended to form a uniform, creamy mixture.

The mixture was applied on lock 1 with an applicator brush, while the hair rested on a sheet of aluminum foil.

The aluminum sheet was then wrapped around the sample and it was left in contact for 30 minutes.

The sample was then washed with Jean Paul Myné® OCRYS Bandha shampoo and rinsed with warm water for 2 minutes.

**Sample 2** (control): the hair sample was bleached with Jean Paul Myné® DOC OLEODEC 30 grams + DOC ENZYME 0.3 30 grams without the addition of the binding mixture.

The bleaching mixtures were applied on the lock with an applicator brush, while the hair rested on a sheet of aluminum foil. The aluminum sheet was then wrapped around the sample and it was left in contact for 30 minutes. Each sample was then washed with Jean Paul Myné® OCRYS Bandha shampoo and rinsed with warm water for 2 minutes.

### Results

There was a noticeable difference between the quality of the hair of lock 1 and that of lock 2 (the control).

Lock 1 was softer, not as kinky, more hydrated and shinier than the control (lock 2).

Both samples were washed and conditioned five more times with the same shampoo and conditioner product.

After five washings, the benefits obtained on lock 1 (treated with the binding mixture + bleaching mixture) were obvious compared with the control (lock 2 - treated with the bleaching mixture alone).

The lock treated with the organic binder exhibited a significant difference in tensile strength and sheen compared to the lock treated with the commercial kit without the organic binder.

Except as defined otherwise, all the technical and scientific terms used here have the same meanings commonly understood by an expert in the sector to which the invention described pertains (cosmetic arts).

The publications and patent documents mentioned in this document are specifically incorporated as references.

Experts in the sector will recognize, or are able to recognize using the normal technique for application of cosmetic products, many equivalent situations to the specific embodiments of the invention described in the above reports (the examples are not exhaustive).

These equivalent applications will be included in the claims attached.

The materials and dimensions of the finding as described above and claimed hereafter, can be of any kind or type depending on needs. Moreover, all the details are replaceable with others that are technically equivalent, without thereby exceeding the scope of protection of this patent application.

## Claims

1. Method for improvement of the esthetic aspect and physical-mechanical characteristics of keratins, **characterized by** the application on them of at least one organic binder, defined as molecular pincers, having the following functional groups:
- two carboxylic functional groups or
- two sulfonic functional groups or
- one carboxylic and one sulfonic functional group,
capable of forming covalent chemical bonds of imidic type with the free primary aminic functional groups of keratins,
wherein the organic binder has the chemical structure indicated by the following Formula I 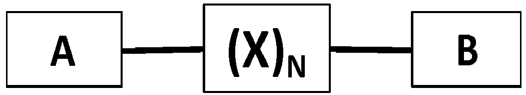 in which:
i. A and B are reactive molecular portions containing, independently: two carboxylic functional groups, or two sulfonic functional groups, or one sulfonic and one carboxylic functional groups, capable of reacting with the free primary aminic functional groups of keratins to form imidic functional groups;
ii. N is a whole number between 0 and 100, preferably between 0 and 50, more preferably between 0 and 10;
iii. X:
- is a single atom, a group of atoms, as well as a functional group (such as -C(=O)-, -CH₂-), or several groups of atoms, such as an alkylenic or polymeric chain;
- includes, but is not limited to Sulfur, Carbon, Boron, Nitrogen or alcoxic, alkylic, alkenylic, cycloalkylic, cycloalkenylic, arylic, heterocyclic alkylic, heteroarylic compounds or ether.

2. Method for the treatment of keratins according to claim 1, **characterized in that** the reactive molecular portion A of the organic binder contains a carboxylic functional group and the reactive molecular portion B of the organic binder contains a sulfonic functional group, or the reactive molecular portion A of the organic binder contains a sulfonic functional group and the reactive molecular portion B of the organic binder contains a carboxylic functional group.

3. Method for the treatment of keratins according to claim 1, **characterized in that** the reactive molecular portions A and B of the organic binder both contain a sulfonic functional group.

4. Method for the treatment of keratins according to claim 1, **characterized in that** the reactive molecular portions A and B of the organic binder both contain a carboxylic functional group.

5. Method for the treatment of keratins according to claims 1 and 2, **characterized in that** a sulfonic functional group and a carboxylic functional group react with an aminic functional group of a keratin fiber 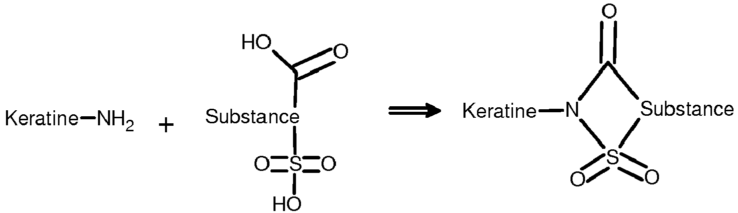 forming a disulfo-carboxy-imidic functional group.

6. Method for the treatment of keratins according to either of claims 1 and 2, **characterized in that** the sulfonic and carboxylic functional groups of two binders react with two aminic groups of two different keratinic fibers 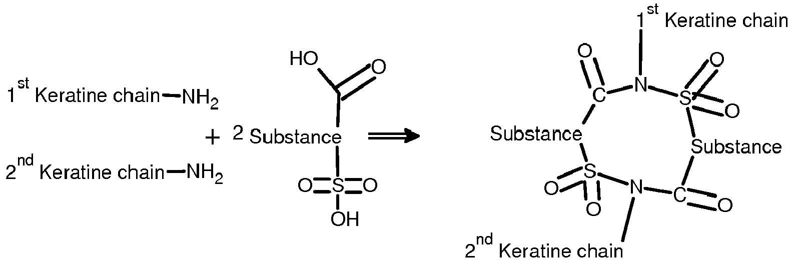 forming disulfo-carboxy-imidic functional groups.

7. Method for the treatment of keratins according to either of claims 1 and 3, **characterized in that** two sulfonic functional groups react with an aminic group of a keratin fiber 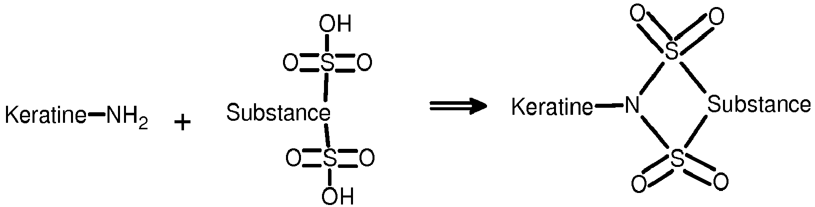 forming a disulfo-imidic functional group.

8. Method for the treatment of keratins according to either of claims 1 and 3, **characterized in that** the sulfonic functional groups of two binders react with the aminic groups of two different keratinic fibers 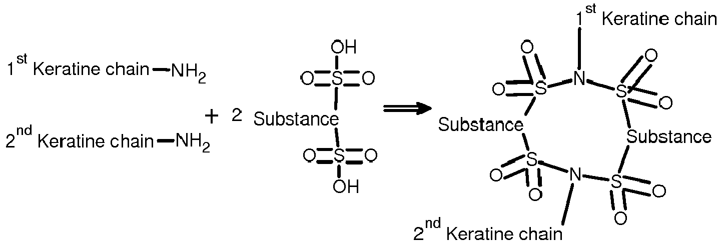 forming disulfo-imidic functional groups.

9. Method for the treatment of keratins according to either of claims 1 and 3, **characterized in that** two carboxylic functional groups react with an aminic group of a keratin fiber 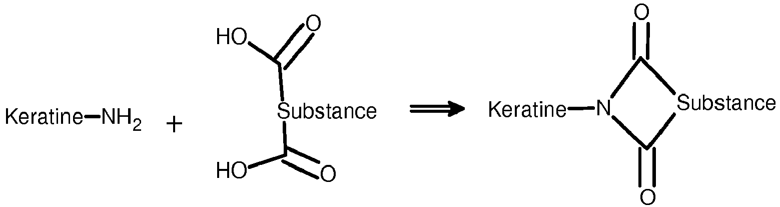 forming a dicarboxy-imidic functional group.

10. Method for the treatment of keratins according to any of claims 1 through 4, **characterized in that** the sulfonic and carboxylic functional groups of two binders react with two aminic groups of two different keratinic fibers 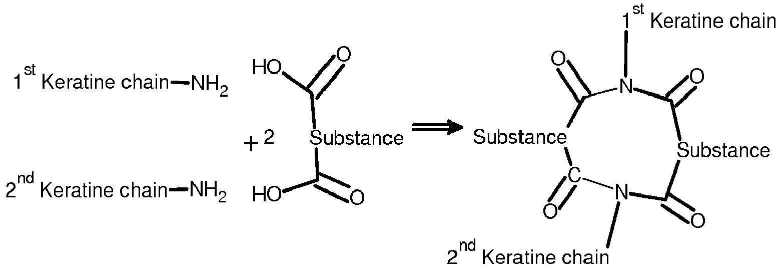 forming dicarboxy-imidic functional groups.

11. Method for the treatment of keratins according to claim 1, **characterized in that** the organic binders according to claims 2, 3 and 4 are dissolved in a cosmetically acceptable medium at a concentration between 0.1% and 99.9 % (weight/weight).

12. Method for the treatment of keratins according to claim 1, **characterized in that** the organic binders according to claims 2, 3 and 4 are used according to the procedure consisting of the following steps:
12.a: application on the keratins of one of the cosmetically acceptable solutions containing organic binders according to claims 2, 3 and 4;
12.b: maintenance in contact of the solution with the keratins for a time between 1 minute and 120 minutes;
12.c: rinsing of the keratins with water;
12.d: drying of the keratins using heated air blowers commonly used in the cosmetic art (hair-dryer);
12.e: possible exposure of the keratins to a heat source that warms to a temperature between 50 and 250°C through the use of tools of common use in the cosmetic arts (curling irons or heating tongs).
